(19) Europäisches Patentamt
European Patent Office
Office européen des brevets

(11) Veröffentlichungsnummer: **0 146 866 B1**

# (12) EUROPÄISCHE PATENTSCHRIFT

(45) Veröffentlichungstag der Patentschrift: **19.06.91**

(51) Int. Cl.⁵: **C07H 15/20**, C12Q 1/34

(21) Anmeldenummer: **84115131.9**

(22) Anmeldetag: **11.12.84**

Die Akte enthält technische Angaben, die nach dem Eingang der Anmeldung eingereicht wurden und die nicht in dieser Patentschrift enthalten sind.

## (54) Phenolsulfonphtaleinyl-Beta-D-galactoside, Verfahren zu deren Herstellung sowie deren Verwendung zur Bestimmung der Beta-D-Galactosidase.

(30) Priorität: **17.12.83 DE 3345748**

(43) Veröffentlichungstag der Anmeldung:
**03.07.85 Patentblatt 85/27**

(45) Bekanntmachung des Hinweises auf die Patenterteilung:
**19.06.91 Patentblatt 91/25**

(84) Benannte Vertragsstaaten:
**AT BE CH DE FR GB IT LI LU NL SE**

(56) Entgegenhaltungen:
**EP-A- 0 060 793**
**EP-A- 0 122 023**
**EP-A- 0 294 804**

**CHEMICAL ABSTRACTS, Band 86, Nr. 17, 25. April 1977, Seite 189, Zusammenfassung Nr. 116822z, Columbus, Ohio, US; & JP-A-76 114 990 (ALZA CORP.) 09-10-1976**

(73) Patentinhaber: **BOEHRINGER MANNHEIM GMBH**
**Sandhofer Strasse 116**
**W-6800 Mannheim 31(DE)**

(72) Erfinder: **Kuhr, Manfred, Dr. rer. nat.**
**Werthmannweg 23**
**W-6800 Mannheim 31(DE)**
Erfinder: **Machat, Rudolf, Dr. rer. nat. dipl. chem.**
**Bassermannstrasse 65**
**W-6800 Mannheim 1(DE)**
Erfinder: **Weckerle, Wolfgang, Dr. rer. nat.**
**Auf dem Leimen 12**
**W-6718 Grünstadt(DE)**
Erfinder: **Batz, Hans-Georg, Dr. rer. nat.**
**Traubinger Strasse 63**
**W-8132 Tutzing(DE)**
Erfinder: **Herrmann, Rupert, Dr. rer. nat.**
**Heinrichstrasse 3**
**W-8120 Weilheim(DE)**
Erfinder: **Kleemann, Wolfgang, Dr. Ph. D.**
**Alpspitzstrasse 7**
**W-8132 Tutzing(DE)**
Erfinder: **Buschek, Herbert**
**Paradeisstrasse 36**
**W-8120 Weilheim(DE)**

**EP 0 146 866 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Oligo- oder Polysaccharide, die D-Galactose mit β-glycosidischer Bindung enthalten, kommen in fast allen Organismen vor. Demgemäß sind auch die entsprechenden β-D-Galactosidasen (EC 3.2.1.23) weit verbreitet und können in zahlreichen Mikroorganismen, Tieren und Pflanzen nachgewiesen werden.

Eine vielfache physiologische Funktion erfüllt die β-D-Galactosidase im Säugetierbereich: Sie spielt eine gewichtige Rolle im Kohlenhydratstoffwechsel, da durch sie die Hydrolyse der Lactose erfolgt. Darüber hinaus stellt die β-D-Galactosidase das Schlüsselenzym beim Abbau von Glycolipiden, Mucopolysacchariden und Glycoproteinen dar.

Über ihren physiologischen Stellenwert hinaus hat die β-D-Galactosidase in den letzten Jahren im diagnostischen Bereich an Bedeutung gewonnen. So wird beispielsweise dieses Enzym in zunehmendem Maße als Indikator-Enzym für Enzymimmunoassays eingesetzt [siehe beispielsweise Annals of Clinical Biochemistry 16, 221 - 240 (1979)].

Die Bestimmung der Aktivität der β-D-Galactosidase spielt demnach sowohl in der klinischen Chemie als auch in der Diagnostik eine zunehmende Rolle. Hierzu wird ganz allgemein die Galactosidase-haltige Probe mit einem geeigneten β-D-Galactosidase-Substrat versetzt. Das Substrat wird von dem Enzym gespalten. Eines der Spaltprodukte wird in geeigneter Weise nachgewiesen. Es kann entweder das durch Einwirkung des Enzyms freigesetzte Glycon oder das Aglycon gemessen werden. In der Regel wird letzteres bestimmt. Als Substrat eignen sich das natürliche Substrat Lactose sowie insbesondere ein chromogenes Galactosid.

So sind in Biochem. Z. 333, 209 (1960) Phenyl-β-D-galactosid sowie einige weitere am aromatischen Ring substituierte Derivate (z. B. o-Nitrophenyl- und p-Nitrophenyl-β-D-galactosid) als Substrate der β-D-Galactosidase beschrieben. Die durch Hydrolyse freigesetzten Phenole werden photometrisch im UV-Bereich bzw. bei den Nitrophenolen im kurzwelligen sichtbaren Wellenlägenbereich bestimmt. Auch kann als Indikatorreaktion eine oxidative Kupplung mit Aminoantipyrin angeschlossen werden [Analytical Biochem. 40, 281 (1971)].

Für histochemische Untersuchungen werden zum einen Naphthyl-β-D-galactoside verwendet: So z. B. die 1-Naphthyl-Verbindung in Histochemie 35, 199 (1973), das 6-Brom-2-naphthyl-Derivat in J. Biol. Chem. 195, 239 (1952) oder das Naphthol-AS-BI-β-D-galactosid [Histochemie 37, 89 (1973)]. Zur Visualisierung werden dabei die entstehenden Naphthole mit verschiedenen Diazoniumsalzen zu Azo-Farbstoffen umgesetzt.

Ferner ist 5-Brom-4-chlor-indoxyl-β-D-galactosid als Substrat der β-Galactosidase bekannt. Indikator-Reaktion ist hier die oxidative Dimerisierung des entstehenden Indoxyls zu Indigo [Histochemie 23, 266 (1970)] oder Kupplung mit Diazoniumsalzen zu Indoxyl-Azo-Farbstoffen [Histochemistry 57, 323 (1978)].

Die vorgestellten Bestimmungsmethoden weisen erhebliche Nachteile auf: Zum einem sind sie zu unempfindlich. Darüber hinaus sind die in den histochemischen Nachweisen eingesetzten Substrate sehr schlecht löslich.

Ferner wurde zum Nachweis von N-Acetyl-β-D-glucosaminidase ein chromogenes Substrat vorgeschlagen, das als farbbildendes Aglykon ein Chromophor der Phenolphthalein-Reihe enthält; siehe EP-A-60 793.

Wesentlich empfindlichere Teste entstehen, wenn als Substrate Galactoside eingesetzt werden, deren Aglykon fluorometrisch nachgewiesen werden kann: So ist in Proc. Nat. Acad. Sci U. S. 47, 1981 (1961) Fluorescein-di-β-D-galactosid als Substrat beschrieben. Außerdem werden 2-Naphthyl-β-D-galactosid [Analytical Biochem. 42, 275 (1971)] oder 4-Methyl-umbelliferyl-β-D-galactosid [Biochem. J. 102, 525 (1967)] verwendet.

Der Nachteil der fluorometrischen Methoden besteht darin, daß hier ein erheblicher apparativer Aufwand betrieben werden muß.

Es bestand daher weiterhin ein Bedarf an Substraten, mit denen die β-D-Galactosidase auf einfache, schnelle und zuverlässige weise bestimmt werden kann. Aufgabe war es, diesen Bedarf zu befriedigen.

Es wurde nun gefunden, daß man β-D-Galactosidase sehr empfindlich im sichtbaren Spektralbereich visuell oder mit einfachen Spektralphotometergeräten nachweisen kann, wenn man Sulfonphthaleinyl-β-D-galactoside als Substrate einsetzt. Diese Verbindungen haben darüber hinaus den Vorteil, daß sie sehr leicht wasserlöslich sind.

Gegenstand der vorliegenden Erfindung sind demnach Sulfonphthaleinyl-β-D-galactoside der allgemeinen Formel I

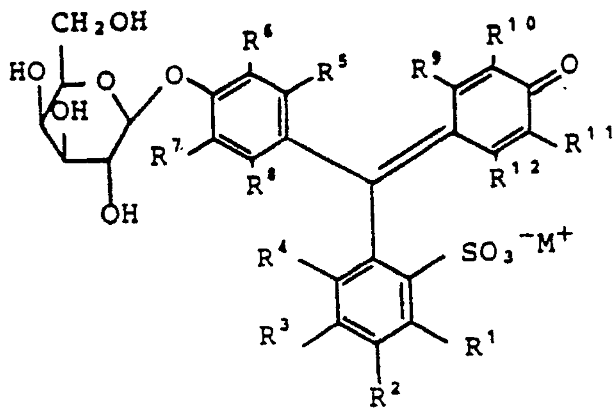

(I)

in der
$R^1$ bis $R^4$, die gleich oder verschieden sein können, jeweils Wasserstoff, Halogen, eine Nitro- oder eine Aminogruppe
$R^5$ bis $R^{12}$, die gleich oder verschieden sein können, jeweils Wasserstoff, Halogen, eine Niederalkyl-, eine Hydroxy-, eine Niederalkoxy-, eine Carboxyl- oder eine Nitrogruppe
$M^+$ ein Proton, ein Alkali-, ein Erdalkali- oder ein Ammoniumion
bedeuten.

Sämtliche Sulfonphthaleinyl-β-D-galactoside der allgemeinen Formel I sind neue Verbindungen. Sie können nach an sich aus der Kohlenhydratchemie her bekannten Methoden hergestellt werden.

Vorzugsweise werden in an sich bekannter Weise Phenolsulfonphthaleine der allgemeinen Formel II,

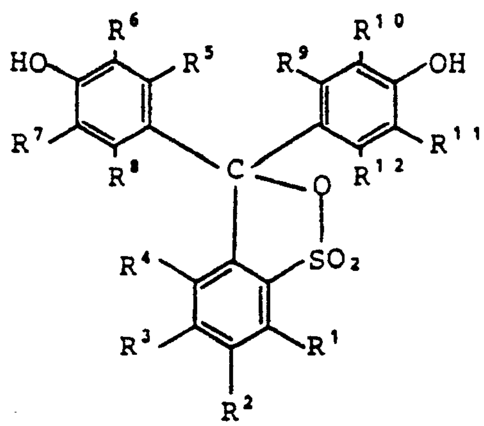

(II)

in der $R^1$ bis $R^{12}$ die oben angegebene Bedeutung haben,
mit per-0-substituierter 1-Halogeno-α-D-galactose der allgemeinen Formel III

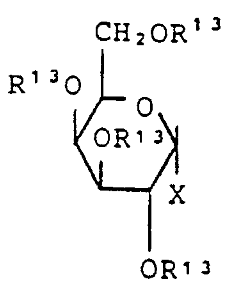

(III)

in der X Halogen und $R^{13}$ eine in der Kohlenhydratchemie gebräuchliche Schutzgruppe bedeuten,
unter Walden-Umkehr am C-1-Atom des Zuckerrestes zu per-0-substituierten Sulfonphthaleinyl-β-D-galactosiden der allgemeinen Formel IV,

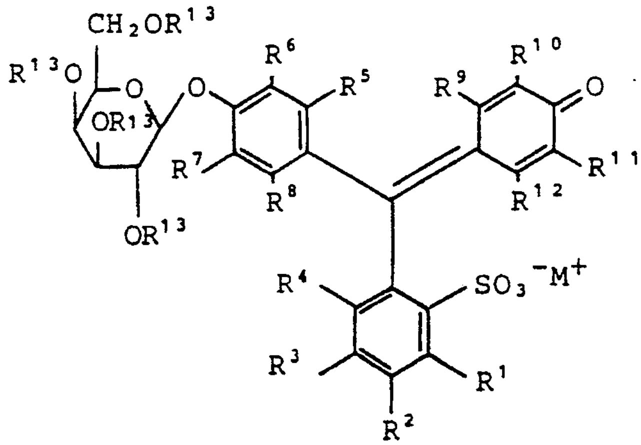

(IV)

umgesetzt und von letzteren in an sich bekannter Weise die Schutzgruppen $R^{13}$ abgespalten.

Die Umsetzung der Verbindungen der Formeln II und III zu Galactosiden der allgemeinen Formel IV wird vorzugsweise in Gegenwart von Säurefängern, wie Alkalihydroxid oder -carbonat, in wäßrigem Aceton oder (unter Phasentransferbedingungen) in einem Wasser/Benzol- oder Wasser/Chloroform-Gemisch vorgenommen.

Desweiteren können die Galactoside der allgemeinen Formel IV hergestellt werden, indem man die Phenolsulfonphthaleine der allgemeinen Formel II zunächst mittels Alkalihydroxid oder -alkoholat in die Di-Alkalisalze bzw. mittels gegebenenfalls substituierter Amine in die Ammoniumsalze überführt und diese dann in dipolar aprotischen Lösungsmitteln wie Aceton, Dimethylsulfoxid, Dichlormethan, Tetrahydrofuran oder Dimethylformamid mit den per-0-substituierten 1-Halogeno-galactosen der allgemeinen Formel III umsetzt.

Ferner haben sich bei der Synthese von Galactosiden der allgemeinen Formel IV aus den Phenolsulfonphthaleinen der allgemeinen Formel II und den 1-Halogeno-galactosen der allgemeinen Formel III Zusätze von einzelnen Silbersalzen oder Gemischen von Silbersalzen (Silberoxid, -carbonat, -carbonat auf Celite, -triflat, -salicylat) und/oder von einzelnen Quecksilbersalzen oder Gemischen von Quecksilbersalzen (Quecksilberbromid, -cyanid, -acetat, -oxid), gegebenenfalls unter Verwendung von Trocknungsmitteln wie Calciumchlorid oder Drierit, in Lösungsmitteln, wie Methylenchlorid, Chloroform, Benzol, Toluol oder Dioxan, bewährt.

Die so erhaltenen per-0-substituierten Sulfonphthaleinyl-$\beta$-D-galactoside der allgemeinen Formel IV sind ebenfalls neue Verbindungen.

Die Abspaltung der Schutzgruppen $R^{13}$ der per-0-substituierten Sulfonphthaleinyl-$\beta$-D-galactoside der allgemeinen Formel IV zu den Sulfonphthaleinyl-$\beta$-D-galactosiden der allgemeinen Formel I wird nach den in der Kohlenhydratchemie gängigen Methoden [s. z. B. Advances Carbohydrate Chem. 12, 157 (1957)], beispielsweise bei den Acyl-Schutzgruppen mittels Natriummethylat oder Bariummethylat oder Ammoniak in Methanol, durchgeführt.

Die Phenolsulfonphthaleine der allgemeinen Formel II sind bekannte handelsübliche Substanzen oder werden nach bekannten Verfahren aus dem entsprechenden Phenol und der entsprechenden o-Sulfonbenzoesäure hergestellt (s. z. B. D. S Breslow und H. Skolnik, in A. Weissberger, The Chemistry of Heterocyclic Compounds, Interscience-Publishers, New York, 1966, Band 21, S. 118) oder werden, ausgehend von bekannten Sulfonphthaleinen, nachtraglich derivatisiert, z. B. halogeniert oder nitriert (vgl. z. B. D. S. Breslow und H. Skolnik, ibid., S. 141; S. 144).

Die als Ausgangsmaterial eingesetzten per-0-substituierten 1-Halogeno-$\alpha$-D-galactosen der allgemeinen Formel III sind ebenfalls bekannte Verbindungen. Sie sind beispielsweise in Chem. Ber. 35, 836 (1902); Nature 165, 369 (1950); Acta chem. Scand., Ser. B, 33, 116 (1979); J. Chem. Soc., 1419 (1965); Carbohydr. Res., 11, 85 (1969) beschrieben.

Unter Halogen in der Definition von $R^1$ bis $R^{12}$ und X ist Fluor, Chlor, Brom und Iod, bei $R^1$ bis $R^{12}$ vorzugsweise Fluor, Chlor und Brom, bei X vorzugsweise Chlor und Brom, zu verstehen.

Die "Niederalkylgruppe" in der Definition von $R^5$ bis $R^{12}$ enthält 1 bis 5, vorzugsweise 1 bis 3 Kohlenstoffatome, wobei die Methyl- und die Isopropylgruppe ganz besonders bevorzugt sind.

Die "Niederalkoxygruppe" in der Definition von $R^5$ bis $R^{12}$ enthält 1 bis 5, vorzugsweise 1 bis 3 Kohlenstoffatome, wobei die Methoxygruppe ganz besonders bevorzugt ist.

Unter "Alkalimetallion" in der Definition von $M^+$ ist das Lithium-, Natrium- und Kaliumion zu verstehen,

wobei das Lithium- und Natriumion bevorzugt sind.

Das "Erdalkaliion" in der Definition von $M^+$ bedeutet Magnesium-, Calcium- und Bariumion, wobei das Calciumion bevorzugt ist.

Unter "Ammoniumion" in der Definition von $M^+$ ist $[N R^{14} R^{15} R^{16} R^{17}]^+$ zu verstehen, wobei $R^{14}$ bis $R^{17}$, die gleich oder verschieden sein können, jeweils Wasserstoff, eine Niederalkylgruppe mit 1 bis 4, vorzugsweise mit 1 oder 2 Kohlenstoffatomen oder eine Benzylgruppe bedeuten.

Als "in der Kohlenhydratchemie gebräuchliche Schutzgruppe" $R^{13}$ eignet sich besonders ein Acetyl-, Benzoyl-, Benzyl- oder Trimethylsilyl-Rest.

Ein weiterer Gegenstand der Erfindung ist die Verwendung der neuen Sulfonphthaleinyl-β-D-galactoside der allgemeinen Formel I zur Bestimmung der Aktivität von β-D-Galactosidase. Ferner werden diagnostische Mittel zur Bestimmung der Aktivität von β-D-Galactosidasen beansprucht, die die neuen Sulfonphthaleinyl-β-D-galactoside der allgemeinen Formel I zur Bestimmung der Aktivität von β-D-Galactosidasen enthalten.

Die Verwendung der Sulfonphthaleinyl-β-D-galactoside als Substrate für die β-D-Galactosidase führt zu deutlich empfindlicheren β-D-Galactosidase-Testsystemen, als sie bisher bekannt sind. Die neuen Substrate können zur Bestimmung der Aktivität von β-D-Galactosidasen mit Vorteil sowohl im biochemischen als auch im klinisch-chemischen Bereich eingesetzt werden. Sie sind empfindlicher. Daraus ergeben sich mehrere Vorteile:

a) Es können geringere β-D-Galactosidase-Aktivitäten gemessen werden.

b) Es können kleinere Probemengen eingesetzt werden.

c) Die Bestimmung der β-D-Galactosidase-Aktivität kann in erheblich kürzerer Zeit erfolgen.

d) Der geringe Probeneinsatz und die günstige Meßwellenlänge vermindern außerdem die Störanfälligkeit der Methode durch andere Probenbestandteile.

Es hat sich gezeigt, daß die beanspruchten Substrate zur Bestimmung der Aktivität von β-D-Galactosidasen jeglicher Herkunft, die sich durchaus in ihrem optimalen pH-Wert unterscheiden können, geeignet sind. Auch in solchen Fällen reagieren diagnostische Mittel mit Substraten der allgemeinen Formel I deutlich empfindlicher als die bisher bekannten Testmittel.

Geeignet sind die Sulfonphthaleinyl-β-D-galactoside der Formel I auch für immunologische Bestimmungsmethoden, bei denen β-D-Galactosidase als Indikator-Enzym verwendet wird, dessen Aktivität nach Durchführung der immunologischen Reaktion ermittelt werden muß. Solche immunologische Bestimmungsmethoden mit enzymatischer Indikatorreaktion sind dem Fachmann als Enzymimmunoassays bekannt. Diese Methoden dienen zur Bestimmung der Konzentration von Proteinen, Polysacchariden, Hormonen, Arzneistoffen und anderen nieder-molekularen Substanzen im Bereich von $10^{-5}$ bis $10^{-12}$ mol/l. Je nach Erfordernis von Phasentrennschritten unterscheidet man zwischen homogener und heterogener Testführung. Eine weitere Unterteilung kann in kompetitive und nicht-kompetitive Testprinzipien erfolgen.

Alle Testprinzipien arbeiten jedoch mit Enzym-Antigen- bzw. Enzym-Antikörper-Konjugaten. Die enzymatische Indikatorreaktion ist allen Enzymimmunoassays gemeinsam.

Ein für solche Zwecke geeignetes Indikatorenzym ist die β-D-Galactosidase. Die Bestimmung der β-D-Galactosidase in solchen Enzymimmunoassays erfolgt üblicherweise, in dem ein geeignetes β-D-Galactosidase-Substrat zugesetzt wird, das enzymatisch gespalten und in üblicher Weise photometrisch vermessen wird.

Eine Verbesserung des β-D-Galactosidase-Testsystems führt demnach ebenfalls zu erheblichen Vorteilen bei solchen Enzymimmunoassays:

1. Die höhere Empfindlichkeit ermöglicht auch hier eine weitere Erniedrigung der Nachweisgrenzen, kürzere Reaktionszeiten und geringeren Probeneinsatz und damit auch geringere Störungen durch andere Probenbestandteile.

2. Die günstigere Meßwellenlänge vermindert bei bestimmten Reaktionsführungen die Störanfälligkeit der Methode durch unlösliche Bestandteile, beispielsweise durch Trübungen.

Das diagnostische Mittel enthält neben einem oder mehreren der erfindungsgemäßen Substrate der allgemeinen Formel I, ein geeignetes Puffersystem sowie gegebenenfalls weitere geeignete, üblicherweise für solche diagnostische Mittel verwendete Zusatzstoffe, wie beispielsweise Netzmittel, Stabilisatoren usw. Das diagnostische Mittel kann in Form einer Lösung, als Lyophilisat, als Pulvergemisch, Reagenstablette oder auf einem saugfähigen Träger aufgezogen, vorliegen.

Das erfindungsgemäße diagnostische Mittel in Form einer Lösung enthält vorzugsweise sämtliche für den Test benötigten Reagentien. Als Lösungsmittel kommen Wasser oder Gemische von Wasser mit einem wasserlöslichen organischen Lösungsmittel, wie z. B. Methanol, Äthanol, Aceton oder Dimethylformamid, in Frage. Aus Haltbarkeitsgründen kann es vorteilhaft sein, die für den Test benötigten Reagentien auf zwei oder mehr Lösungen zu verteilen, die erst bei der eigentlichen Untersuchung vermischt werden.

Zur Herstellung des diagnostischen Mittels in Form eines Lyophilisats im Gesamtgewicht von jeweils etwa 5 - 20 mg, vorzugsweise etwa 10 mg, wird eine Lösung getrocknet, die neben sämtlichen für den Test benötigten Reagentien übliche Gerüstbildner, wie z. B. Polyvinylpyrrolidon, und evtl. weitere Füllstoffe, wie z. B. Mannit, Sorbit oder Xylit enthält.

Ein diagnostisches Mittel in Form von Pulvermischungen oder Reagenstabletten läßt sich herstellen, indem die Bestandteile des Testes mit üblichen galenischen Zusatzstoffen versetzt und granuliert werden. Zusatzstoffe dieser Art sind z. B. Kohlenhydrate, wie z. B. Mono-, Oligo- oder Polysaccharide, oder Zuckeralkohole, wie z. B. Mannit, Sorbit oder Xylit, oder andere lösliche inerte Verbindungen, wie Polyethylenglykole oder Polyvinylpyrrolidon. Die Pulvermischungen oder Reagenstabletten weisen im allgemeinen ein Endgewicht von ungefähr 30 bis 200 mg, vorzugsweise 50 bis 80 mg, auf.

Zur Herstellung des diagnostischen Mittels in Form eines Teststreifens wird ein saugfähiger Träger, vorzugsweise Filterpapier, Cellulose oder Kunstfaservlies mit Lösungen der erforderlichen, üblicherweise zur Herstellung von Teststreifen verwendeten Reagentien in leicht flüchtigen Lösungsmitteln, wie z. B. Wasser, Methanol, Äthanol oder Aceton imprägniert. Dies kann in einem Imprägnierungsschritt erfolgen. Oft ist es jedoch zweckmäßig, die Imprägnierung in mehreren Schritten durchzuführen, wobei Lösungen eingesetzt werden die jeweils einen Teil der Bestandteile des diagnostischen Mittels enthalten. So kann beispielsweise in einem ersten Schritt mit einer wäßrigen Lösung , die den Puffer und andere wasserlösliche Zusatzsstoffe enthält, und dann in einem zweiten Schritt mit einer Lösung, die die $\beta$-D-Galactosidase-Substrate enthält, imprägniert werden. Die fertigen Testpapiere können als solche verwendet werden oder in an sich bekannter Weise an Griffen angeklebt oder vorzugsweise zwischen Kunststoffen und feinmaschigen Netzwerken gemäß DBP 2118455 eingesiegelt werden.

Die nachfolgenden Beispiele zeigen einige der zahlreichen Verfahrensvarianten, die zur Synthese der erfindungsgemäßen Verbindungen beschritten werden können sowie beispielhaft die Verwendung der neuen Sulfonphthaleinyl-$\beta$-D-galactoside zur Bestimmung der Aktivität von $\beta$-D-Galactosidase. Sie sollen jedoch nicht eine Einschränkung des Erfindungsgegenstandes darstellen.

Es werden die folgenden Abkürzungen verwendet:

| | |
|---|---|
| HEPES | 2-[4-(2-Hydroxyethyl)-1-piperazinyl]-ethansulfonsäure |
| BSA | Rinderserum-Albumin, (bovineserum-albumin) |
| Tween-20 | Polyoxyethylen(20)sorbitanmonolaurat |
| Tricin | [N-Tris(hydroxymethyl)]methyl-glycin |

Beispiel 1

3,3'-Dichlor-phenolsulfonphthaleinyl-$\beta$-D-galactosid-natriumsalz

a) Eine Lösung von 45 g (0.11 Mol) 2,3,4,6-Tetra-O-acetyl-$\alpha$-D-galactopyranosylbromid in 450 ml Chloroform wird auf 60 °C erwärmt. Unter Rühren werden bei dieser Temperatur zugegeben: eine Lösung von 29.9 g (0.11 Mol) Benzyltriethylammoniumbromid in 114 ml 1.25 N wäßriger Natronlauge (0.142 Mol), danach 46.5 g (0.11 Mol) 3,3'-Dichlor-phenolsulfonphthalein (Chlorphenolrot). Reste des Farbstoffes werden von der Gefäßwand mit etwas Wasser und weiteren 114 ml 1.25 N wäßriger Natronlauge heruntergespült.

Das Reaktionsgemisch wird 12 Stunden am Rückfluß gekocht, danach 8 Stunden bei Raumtemperatur stehengelassen. Die organische Phase wird abgetrennt, die wäßrige Phase wird mehrmals mit Chloroform geschüttelt.

Zur Entfernung noch vorhandenen Ausgangsmaterials werden die vereinigten organischen Phasen mehrmals mit 0.1 N wäßriger Natronlauge geschüttelt.

Nach Waschen der Chloroformphase mit Wasser und Trocknen mit Natriumsulfat wird das organische Lösungsmittel eingeengt. Der Rückstand wird mit Ether angerieben und ergibt

46 g 3,3'-Dichlor-phenolsulfonphthaleinyl-2,3,4,6-tetra-O-acetyl-$\beta$-D-galactosid-natriumsalz

gelbes amorphes Material (Ausbeute: 54 % d. Th.) Fp. 190 °C (Zers.)

NMR: (DMSO-$d_6$): 1.95 (s, 3H), 1.99 (s, 3H), 2.02 (s, 3H), 2.12 (s, 3H), 4.0 - 4.6 (m, 4H), 5.1 - 5.7

(m, 3H), 6.1 - 6.8 (m, 1H), 6.9 - 7.7 (m, 8H), 7.8 - 8.0 (m,1H)

b) Eine Lösung von 28 g (0.036 Mol) des nach a) hergestellten Tetraacetylgalactosids in 270 ml absolutem Methanol wird auf 0 - 5 °C gekünlt. Zur Desacetylierung werden unter Rühren bei dieser Temperatur 72 ml einer 1 molaren (0.072 Mol) Natriummethylat-Lösung in Methanol zugesetzt.

Nach 15 Minuten bei 0 - 5 °C wird die Lösung zur Entfernung der überschüssigen Natriumionen mit ca. 300 ml Amberlite IRC 50 versetzt und das Gemisch 2 Stunden bei 5 °C gerührt. Nach Absaugen des Ionenaustauschers wird dieser mehrfach mit Methanol gewaschen.

Nach Einengen der vereinigten Filtrate wird der Rückstand säulenchromatographisch an Kieselgel mit Methylenchlorid/Methanol = 5/1 gereinigt und ergibt 12 g 3,3'-Dichlor-phenolsulfonphthaleinyl-β-D-galactosid-natriumsalz

gelbes, amorphes Pulver (Ausbeute: 55 % d. Th.) Fp. 210 °C (Zers.)

NMR: (DMSO-$d_6$): 3.3 - 3.7 (m, 6H), 3.9 - 5.0 (m, 4H), 5.1 (d, J = 7 Hz, 1H), 6.1 - 6.8 (m, 1N), 6.9 - 7.6 (m, 8H), 7.8 - 8.0 (m, 1H).

## Beispiel 2

In analoger Weise, wie in Beispiel 1 beschrieben werden durch Umsetzung von 2,3,4,6-Tetra-0-acetyl-α-D-galactopyranosylbromid mit den unten unter "Ausgangsmaterial" aufgeführten Phenolsulfonphthaleinen über die entsprechenden peracetylierten Galactoside die unter "Endprodukt" erwähnten β-D-Galactoside hergestellt:

| Ausgangsmaterial | Endprodukt | Fp [°C] |
|---|---|---|
| 1) Phenolrot | Phenolsulfonphthaleinyl-ß-D-galactosid-natriumsalz | 218-220 |
| 2) Fluorphenolrot | 3,3'-Difluorphenolsulfonphthaleinyl-ß-D-galactosid-natriumsalz | glasartig |
| 3) Chlorphenolblau | 3,3',5,5'-Tetrachlorphenolsulfonphthaleinyl-ß-D-galactosid-natriumsalz | 145-150 |

| | | | |
|---|---|---|---|
| 4) | Brenzcatechin violett | 3,3'-Dihydroxyphenolsulfonphthaleinyl-ß-D-galactosid-natriumsalz | 115-120 |
| 5) | Jodphenolblau | 3,3',5,5'-Tetraiodphenolsulfonphthaleinyl-ß-D-galactosid-natriumsalz | 210-215 |
| 6) | m-Cresolpurpur | 2,2'-Dimethylphenolsulfonphthaleinyl-ß-D-galactosid-natriumsalz | 205-209 |
| 7) | Bromcresolpurpur | 3,3'-Dibrom-5,5'-dimethylphenolsulfonphthaleinyl-ß-D-galactosid-natriumsalz | 200-203 |
| 8) | o-Cresolrot | 3,3'-Dimethylphenolsulfonphthaleinyl-ß-D-galactosid-natriumsalz | 200-204 |
| 9) | Thymolblau | 3,3'-Diisopropyl-6,6'-dimethylphenolsulfonpthaleinyl-ß-D-galactosid-natriumsalz | 205-209 |
| 10) | Bromthymol blau | 3,3'-Dibrom-5,5'-diisopropyl-2,2'-dimethylphenolsulfonphthaleinyl-ß-D-galactosid-natriumsalz | 190-195 |
| 11) | Salicylrot | 3,3'-Dicarboxyphenolsulfonphthaleinyl-ß-D-galactosid-natriumsalz | 178-180 |

| | | | |
|---|---|---|---|
| 12) | 3,3',5,5'-Tetrabrom-2,2'-dimethylphenolsulfonphthalein | 3,3',5,5'-Tetrabrom-2,2'-dimethylphenolsulfonphthaleinyl-ß-D-galactosid-natriumsalz | 100-103 |
| 13) | 3,3'-Dinitrophenolsulfonphthalein | 3,3'-Dinitrophenolsulfonphthaleinyl-ß-D-galactosid-natriumsalz | 167-170 |
| 14) | 3,3'-Dichlor-5,5'-dinitrophenolsulfonphthalein | 3,3'-Dichlor-5,5'-dinitrophenol-sulfonphthaleinyl-ß-D-galactosid-natriumsalz | 115-118 |
| 15) | 3,3'-Dimethyl-5,5'-dinitrophenolsulfonphthalein | 3,3'-Dimethyl-5,5'-dinitrophenolsulfonphthaleinyl-ß-D-galactosid-natriumsalz | 155-158 |
| 16) | 3,3'-Dimethoxyphenolsulfonphthalein | 3,3'-Dimethoxyphenolsulfonphthaleinyl-ß-D-galactosid | |
| 17) | 3,3'-Difluorphenyl-3",4",5",6"-tetrabromsulfonphthalein | 3,3'-Difluorphenyl-3",4",5",6"-tetrabromsulfonphthaleinyl-ß-D-galactosid | |
| 18) | 2,2'-Dimethyl-3,3'-dinitrophenolsulfonphthalein | 2,2'-Dimethyl-3,3"-dinitrophenolsulfonphthaleinyl-ß-D-galactosid | |

| | | | |
|---|---|---|---|
| 19) | 2,2'-Dimethyl-5,5'-dinitrophenolsulfonphthalein | 2,2'-Dimethyl-5,5'-dinitrophenolsulfonphthaleinyl-ß-D-galactosid | |
| 20) | Phenol-4"-nitrosulfonphthalein | Phenol-4"-nitrosulfonphthaleinyl-ß-D-galactosid natriumsalz | 300 |
| 21) | Phenol-5"-nitrosulfon phthalein | Phenol-5"-nitrosulfonphthaleinyl-ß-D-galactosid | |
| 22) | 3,3'-Dichlorphenol-4"-nitrosulfonphthalein | 3,3'-Dichlorphenol-4"-nitrosulfonphthaleinyl-ß-D-galactosid-natriumsalz | 160/ 300 |
| 23) | 3,3'-Difluorphenol-4"-nitrosulfonphthalein | 3,3'-Difluorphenol-4"-nitrosulfonphthaleinyl-ß-D-galactosid | |
| 24) | 3,3',4"-Trinitrophenolsulfonphthalein | 3,3'4"-Trinitrophenolsulfonphthaleinyl-ß-D-galactosid natriumsalz | 300 |
| 25) | Phenol-4"-aminosulfonphthalein | Phenol-4"-aminosulfonphthaleinyl-ß-D-galactosid-natriumsalz | amorph |

Beispiel 3

3,3'-Di-fluor-phenolsulfonphthaleinyl-β-D-galactosid-natriumsalz

a) 6.2 g (0.016 Mol) 3,3'-Di-fluor-phenolsulfonphthalein (Fluorphenolrot) werden in 170 ml absolutem Methanol gelöst. Zur Bildung des Di-natriumsalzes werden 32 ml (0.032 Mol) einer 1 molaren Natriummethylatlösung in Methanol zugefügt. Die Lösung wird zur Trockne eingeengt. Zur Lösung des Rückstandes in 140 ml absolutem Dimethylformamid gibt man 7.3 g (0.0176 Mol) 2,3,4,6-Tetra-O-acetyl-α-D-galactopyranosylbromid hinzu und rührt 6 Stunden bei Raumtemperatur. Nach Absaugen wird das Filtrat bei Raumtemperatur im Ölpumpenvakuum eingeengt. Der Rückstand wird mit Ether angerieben, abgesaugt und getrocknet und ergibt
6.9 g 3,3'-Di-fluor-phenolsulfonphthaleinyl-2,3,4,6-tetra-0-acetyl-β-D-galactosid-natriumsalz
orangefarbenes amorphes Material (Ausbeute 63 % d. Th.) Fp. 215 °C (Zers.)
NMR: (DMSO-$d_6$): 1.94 (s, 3H), 1.96 (s, 3H), 1.99 (s, 3H), 2.15 (s, 3H), 3.9 - 4.7 (m, 4H), 5.0 - 5.6 (m, 3H), 6.2 - 6.6 (m, 1H), 7.0 - 7.6 (m, 8H), 7.9 - 8.2 (m, 1H)

b) Eine Lösung von 3.5 g (0.005 Mol) des nach a) hergestellten Tetraacetylgalactosids in 750 ml absolutem Methanol wird bei Raumtemperatur versetzt mit 1.5 ml (0.0015 Mol) einer 1 molaren Natriummethylatlösung in Methanol. Nach Stehen über Nacht wird die Lösung eingeengt. Der Rückstand wird säulenchromatographisch an Kieselgel mit Methylenchlorid/Methanol = 5/1 gereinigt.
Man erhält
1.2 g 3,3'-Difluor-phenolsulfonphthaleinyl-β-D-galactosid-natriumsalz
orangerotes, hygroskopisches, amorphes Pulver (Ausbeute: 41 % d. Th)
NMR: (DMSO-$d_6$): 3.1 - 3.9 (m, 6H), 4.1 - 5.3 (m, 4H) 4.95 (d, J = 7 Hz, 1H), 6.2 - 6.6 (m, 1H), 6.8 - 7.6 (m, 8H), 7.8 - 8.0 (m, 1H).

Beispiel 4

In analoger weise, wie im vorangehenden Beispiel 3 ausgeführt, werden aus 2,3,4,6-Tetra-0-acetyl-α-D-galactopyranosylbromid und den unten unter "Ausgangsmaterial" aufgeführten Phenolsulfonphthaleinen die unter "Endprodukt" erwähnten β-D-Galactoside dargestellt:

| Ausgangsmaterial | Endprodukt | Fp °C |
|---|---|---|
| 1) Phenolrot | Phenolsulfonphthaleinyl-ß-D-galactosid-natriumsalz | 208-212 |
| 2) o-Cresolrot | 3,3'-Dimethylphenolsulfonphthaleinyl-ß-D-galactosid-natriumsalz | 202-205 |
| 3) Bromcresolpurpur | 3,3'-Dibrom-5,5'-dimethylphenolsulfonphthaleinyl-ß-D-galactosid-natrium·alz | 198-202 |

Beispiel 5

3,3'-Dibrom-5,5'-dimethylphenolsulfonphthaleinyl-β-D-galactosid-natriumsalz

a) Eine Lösung von 11.03 g (0.027 Mol) 2,3,4,6-Tetra-0-acetyl-α-D-galactopyranosylbromid und 5.6 g (0.007 Mol) Bromcresolpurpur-tribenzylammoniumsalz in 60 ml Dichlormethan werden mit 3.1 g (0.013 Mol) Silberoxid und 3.7 g (0.013 Mol) Silbercarbonat versetzt und 12 Stunden bei Raumtemperatur

gerührt. Nach Abfiltrieren des Niederschlages wird das Filtrat eingeengt und der Rückstand säulenchromatographisch an Kieselgel mit Toluol/Essigester/Methanol = 1/1/0.2 gereinigt. Einengen der entsprechenden Fraktion ergibt

4.4 g 3,3'-Dibrom-5,5'-dimethylphenolsulfonphthaleinyl-2,3,4,6-tetra-0-acetyl-β-D-galactosid-tribenzylammoniumsalz

gelbes, amorphes Material (Ausbeute 54 % d. Th.)

NMR: ($DMSO-d_6$): 1.8 - 2.3 (m, 18H), 3.8 - 4.4 (m, 4H), 5.2 - 5.6 (m, 9 H), 6.6 - 8.1 (m, 23 H).

b) Eine Lösung von 4 g (0.0035 Mol) des nach a) hergestellten Tetraacetylgalactosids in 40 ml absolutem Methanol wird auf -40° C gekühlt und zur Desacetylierung mit 15.5 ml einer 1 M (0.015 Mol) Natriummethylat-Lösung versetzt.

Nach einer Stunde wird die Lösung durch Behandlung mit ca. 30 ml Amberlite IRC 50 (H-Form) neutralisiert und eingeengt. Der Rückstand wird säulenchromatographisch an Kieselgel mit Methylenchlorid/Methanol/Aceton = 6/2/1 gereinigt und ergibt

2 g 3,3'-Dibrom-5,5'-dimethylphenolsulfonphthaleinyl-β-D-galactosid-natriumsalz

gelbes, amorphes Pulver (Ausbeute 62 % d. Th.) Fp. 200 - 203° C (Zers.)

NMR: ($DMSO-d_6$): 1.9 - 2.4 (m, 6H), 3.2 - 4.0 (m, 6H), 4,4 (m, 2H), 4.8 (m, 2H) [nach Deuteriumaustausch: 4.9 (d, J = 7 Hz, 1H)], 6.7 - 8.1 (m, 8H).

<u>Beispiel 6</u>

In analoger Weise, wie im vorstehenden Beispiel 5 beschrieben, werden hergestellt aus 2,3,4,6-Tetra-0-acetyl-α-D-galactopyranosylbromid und

1) Fluorphenolrot
das <u>3,3'-Difluorphenolsulfonphthaleinyl-β-D-galactosid-natriumsalz</u>
hygroskopisches Glas

2) Phenol-3",4",5",6"-tetrabromsulfonphthalein
das <u>Phenol-3",4",5",6"-tetrabromsulfonphthaleinyl-β-D-galactosid-natriumsalz,</u>
Fp 215° C (Zers.)

3) 3,3',5,5'-Tetrachlorphenol-3",4",5",6"-tetrabromsulfonphthalein
das <u>3,3',5,5'-Tetrachlorphenol-3",4",5",6"-tetrabromsulfonphthaleinyl-β-D-galactosid-natriumsalz,</u>
Fp. 150° C (Zers.)

4) Phenol-4"-nitrosulfonphthalein
das <u>Phenol-4"-nitrosulfonphthaleinyl-β-D-galactosid-natriumsalz</u>
Fp > 300° C

5) 3,3'-Dichlorphenol-4"-nitrosulfonphthalein
das <u>3,3'-Dichlorphenol-4"-nitrosulfonphthaleinyl-β-D-galactosid-natriumsalz</u>
Doppel-Fp 160° C/ >300° C

6) 3,3'-Dimethylphenol-4"-nitrosulfonphthalein
das <u>3,3'-Dimethylphenol-4"-nitrosulfonphthaleinyl-β-D-galactosid-natriumsalz</u>
Fp 210 - 220° C

7) 3,3',4"-Trinitrophenolsulfonphthalein
das <u>3,3',4"-Trinitrophenolsulfonphthaleinyl-β-D-galactosid-natriumsalz</u>
Fp >300°

8) Phenol-4"-aminosulfonphthalein
das <u>Phenol-4"-aminosulfonphthaleinyl-β-D-galactosid-natriumsalz</u>
Lyophilisat (amorph)

<u>Beispiel 7</u>

<u>3,3'-Difluorphenolsulfonphthaleinyl-β-D-galactosid-tribenzylammoniumsalz</u>

Eine Lösung von 6.77 g (0.01 Mol) Fluorphenolrot-tribenzylammoniumsalz und 5.3 g (0.01 Mol) Per-O-trimethylsilyl-α-D-galactopyranosylbromid in 70 ml Dichlormethan werden mit 1.15 g (0.005 Mol) Silberoxid und 1.4 g (0.005 Mol) Silbercarbonat versetzt und 18 Stunden unter Feuchtigkeitsausschluß gerührt. Nach Abfiltrieren des Niederschlags wird das Filtrat eingeengt, der Rückstand zum Abspalten der Schutzgruppen in 60 ml Methanol aufgenommen und 12 Stunden bei Raumtemperatur aufbewahrt. Zur Reinigung wird mit Dichlormethan/Methanol = 5/1 über Kieselgel chromatographiert. Man erhält

1.2 g 3,3'-Difluorphenolsulfonphthaleinyl-β-D-galactosid-tribenzylammoniumsalz

orangefarbenes, amorphes Pulver (Ausbeute 14 % d. Th.)
Fp. 157 - 165 °C
NMR: (DMSO-$d_6$): 3.1 - 3.9 (m, 6H), 4.2 - 5.2 (m, 11H), 6.3 (m, 1H), 6.8 - 7.6 (m, 23H), 7.9 (m, 1H).

Beispiel 8

3,3'-Dichlor-phenolsulfonphthaleinyl-β-D-galactosid-lithiumsalz

1.5 g (0.0025 Mol) 3,3'-Dichlor-phenolsulfonphthaleinyl-β-D-galactosid-natriumsalz, hergestellt nach Beispiel 1, werden in wenig Wasser gelöst. Die Lösung wird auf eine mit Amberlite IR 120 (Li-Form) gefüllte Säule gegeben. Lyophilisierung des Eluates liefert
1.4 g 3,3'-Dichlor-phenolsulfonphthaleinyl-β-D-galactosid-lithiumsalz
orangefarbenes, amorphes Pulver (Ausbeute: 96 % d. Th.)
Fp. 190 °C (Zers.)
NMR: (DMSO-$d_6$): 3.3 - 3.8 (m, 6H), 4.3 - 4.9 (m, 4H), 5.07 (s, J = 7 Hz, 1H), 6.1 - 7.7 (m, 9H), 7.8 - 8.1 (m, 1H).

Beispiel 9

In analoger Weise, wie im vorstehenden Beispiel 8 ausgeführt, werden hergestellt aus
3,3'-Dichlorphenolsulfonphthaleinyl-β-D-galactosid-natriumsalz
(s. Beispiel 1)
a) durch Austauschchromatographie an Amberlite IR 120 in der Ca-Form das
3,3'-Dichlor-phenolsulfonphthaleinyl-β-D-galactosid-calciumsalz
orangerotes, amorphes Produkt (Ausbeute: 78 % d. Th.)
Fp. 250 °C (Zers.)
NMR: (DMSO-$d_6$): 3.2 - 3.8 (m, 6H), 4.4 - 5.1 (m, 4H), 5.1 (s, J = 7Hz, 1 H), 6.2 - 7.6 (m, 9H), 7.8 - 8.0 (m, 1H).
b) Durch Austauschchromatographie an Amberlite IR 120 in der $(H_3C)_4N$-Form das
3,3'-Dichlorphenolsulfonphthaleinyl-β-D-galactosid-tetramethylammoniumsalz
gelbes, amorphes Produkt (Ausbeute 85 % des Th.)
Fp. 190 - 195 °C
NMR: (DMSO-$d_6$): 3.2 (s, 12 H), 3.3 - 4.0 (m, 6H), 4.1 - 5.3 (m, 4H), 5.1 (d, J = 7 Hz, 1H), 6.4 (m, 1H), 7.0 - 7.7 (m, 8H), 7.9 (m, 1H)

Beispiel 10

3,3'-Dichlorphenolsulfonphthaleinyl-β-D-galactosid-tribenzylammoniumsalz

1.5 g (0.0025 Mol) 3,3'-Dichlorphenolsulfonphthaleinyl-β-D-galactosid-natriumsalz (s. Beispiel 1) wird in wenig Wasser gelöst und durch eine Säule, gefüllt mit Amberlite IR 120 (H-Form), passiert. Das Eluat wird mit einer stöchiometrischen (0.72 g) Menge Tribenzylamin, gelöst in 15 ml Ethanol, versetzt und eingeengt. Man erhält
1.7 g 3,3'-Dichlorphenolsulfonphthaleinyl-β-D-galactosid-tribenzylammoniumsalz
gelbes, amorphes Material (Ausbeute 78 % d. Th.)
Fp. 140 - 150 °C

Beispiel 11

In analoger Weise, wie in Beispiel 10 beschrieben, wird hergestellt aus 3,3'-Dichlorphenolsulfonphthaleinyl-β-D-galactosid-natriumsalz (s. Beispiel 1) unter Verwendung von Benzyldiethylamin das entsprechende

3,3'-Dichlorphenolsulfonphthaleinyl-β-D-galactosid-benzyldiethylammoniumsalz

gelbes, amorphes Pulver (Ausbeute 69 % d. Th.)
Fp. 245 - 248 °C

Beispiel 12

Bestimmung der Aktivität der β-D-Galactosidase

a) Zubereitung der verwendeten Lösungen:

| | | |
|---|---|---|
| Pufferlösung: | HEPES | 100 mmol/l |
| | Natriumchlorid | 154 mmol/l |
| | Magnesium-L-Aspartat | 2 mmol/l |
| | BSA | 10 g/l |
| | Tween-20 | 0,5 g/l |
| | pH-Wert (mit Natronlauge eingestellt) | 7,3 (37 °C) |

Reagenslösung 1:

In der vorstehend beschriebenen Pufferlösung werden 5 mmol/l Phenolsulfonphthaleinyl-β-D-galactosid-natriumsalz gelöst. Der pH-Wert wird mit Natronlauge auf pH 8,5 (37 °C) eingestellt.

Reagenslösung 2:

In der vorstehend beschriebenen Pufferlösung werden 5 mmol/l 3,3'-Di-fluor-phenolsulfonphthaleinyl-β-D-galactosid-natriumsalz gelöst. Der pH-Wert wird mit Natronlauge auf pH 7,5 (37 °C) eingestellt.

Reagenslösung 3:

In der vorstehend beschriebenen Pufferlösung werden 5 mmol/l 3,3'-Dichlor-phenolsulfonphthaleinyl-β-D-galactosid-natriumsalz gelöst. Der pH-Wert der Pufferlösung von 7,3 (37 °C) wird beibehalten.

Reagenslösung 4:

In der vorstehend beschriebenen Pufferlösung werden 5 mmol/l 3,3',5,5'-Tetrachlorphenol-3",4",5",6"-tetrabromsulfonphthaleinyl-β-D-galactosid-natriumsalz gelöst. Der pH-Wert der Pufferlösung von 7.3 (37 °C) wird beibehalten.

Die Substratkonzentration und pH-Werte sind für jedes eingesetzte Substrat zu optimieren. Es können daher ganz bewußt unterschiedliche Werte für Substratkonzentrationen bzw. pH-Werte bei den einzelnen Reagenslösungen auftreten.

Enzymlösung

Handelsübliche β-D-Galactosidase aus Escherichia coli wird in der vorstehend genannten Pufferlösung gelöst. Die Aktivität dieser Lösung beträgt ca. 0,08 U/ml (bezogen auf die Hersteller-Angaben).

b) Durchführung der Messungen

Die Messung erfolgt photometrisch bei der jeweils unten angegebenen Wellenlänge.

950 µl Reagens werden in einer 1-cm-Küvette bei 37 °C mit 50 µl Enzymlösung vermischt. Als Maß für die Reaktion wird der Extinktionsanstieg pro Zeiteinheit in [mExt/min] ermittelt. Er wird berechnet aus der gemessenen Extinktion durch Division mit der Reaktionszeit.

In der folgenden Tabelle sind die gefundenen Meßwerte aufgeführt:

| Reagens Nr. | Meßwellenlänge [nm] | Reaktion [mExt/min] |
|---|---|---|
| 1 | 560 | 9 |
| 2 | 578 | 71 |
| 3 | 578 | 123 |
| 4 | 578 | 121 |

Beispiel 13

Bestimmung der Aktivität der β-D-Galactosidase

a) Zubereitung der verwendeten Lösungen

| | | |
|---|---|---|
| Pufferlösung: | HEPES | 50 mmol/l |
| | Citronensäure | 50 mmol/l |
| | Tricin | 50 mmol/l |
| | Natriumchlorid | 154 mmol/l |
| | Magnesium-L-Aspartat | 1 mmol/l |
| | BSA | 10 g/l |
| | pH-Wert (mit Natronlauge eingestellt) | 6,9 (37°C) |

Reagenslösung 1:

In vorstehend beschriebener Pufferlösung werden 5 mmol/l 3,3'-Di-methyl-phenolsulfonphthaleinyl-β-D-galactosid gelöst.

Reagenslöstung 2:

In vorstehend beschriebener Pufferlösung werden 5 mmol/l 3,3'-Di-hydroxy-phenolsulfonphthaleinyl-β-D-galactosid gelöst.

Enzymlösung:

Handelsübliche β-D-Galactosidase aus Escherichia coli wird in der vorstehend genannten Pufferlösung gelöst. Die Aktivität dieser Lösung beträgt ca. 0,08 U/ml (bezogen auf die Hersteller-Angaben).

b) Durchführung der Messungen

950 µl Reagens werden in einer 1-cm-Küvette bei 37° C mit 50 µl Enzymlösung vermischt. Nach 10

Minuten Reaktionszeit wird mit Natronlauge auf pH 10 eingestellt und die Extinktion gemessen. Mit einer Blindprobe mit Puffer anstelle von Enzymlösung wird in gleicher Weise verfahren. Die Temperatur wird während der Reaktion und der Messung auf 37 °C gehalten. Aus den ermittelten Extinktionen der Ansätze mit und ohne Enzym wird die Extinktionsdifferenz errechnet. Durch Division dieser Extinktionsdifferenz durch die Reaktionszeit ergibt sich als Maß für die Reaktion der Extinktionsanstieg pro Zeiteinheit in [mExt/min]

In der folgenden Tabelle sind die gefundenen Meßwerte aufgeführt:

| Reagens Nr. | Meßwellenlänge [nm] | Reaktion [mExt/min] |
|---|---|---|
| 1 | 578 | 98 |
| 2 | 595 | 6 |

Beispiel 14

Bestimmung der Aktivität von β-D-Galactosidasen unterschiedlicher Herkunft

Verwendet werden handelsübliche β-D-Galactosidase-Präparate unterschiedlicher Herkunft. Diese entfalten als charakteristisches Merkmal ihre max. Aktivität bei unterschiedlichen pH-Werten.

Angaben der Hersteller:

| | |
|---|---|
| aus Bohnen (Jack Beans) | pH 3,5 |
| aus Aspergillus niger | pH 4,0 |
| aus Escherichia coli | pH 6,9 |
| aus Rinderleber | pH 7,3 |

a) Herstellung der verwendeten Lösungen:

| | | |
|---|---|---|
| Pufferlösungen: | HEPES | 50 mmol/l |
| | Citronensäure | 50 mmol/l |
| | Tricin | 50 mmol/l |
| | Natriumchlorid | 154 mmol/l |
| | Magnesium-L-Aspartat | 1 mmol/l |
| | BSA | 10 g/l |

Es werden Lösungen obiger Zusammensetzung mit unterschiedlichen pH-Werten gemäß den pH-Optima der β-D-Galactosidasen hergestellt (pH 3,5/4,0/6,9/7,3). Die Einstellung der pH-Werte erfolgt mit NaOH bzw. HCl bei 37 °C.

Reagenslösungen:

In den vorstehend genannten Pufferlösungen mit den unterschiedlichen pH-Werten 3,5/4,0/6,9/7,3 werden jeweils 5 mmol/l 3,3'-Dichlor-phenolsulfonphthaleinyl-β-D-galactosid-natriumsalz gelöst.

Enzymlösungen:

Die β-D-Galactosidasen werden in den Puffern mit den jeweils optimalen pH-Werten gelöst:

| | |
|---|---|
| aus Bohnen (Jack Beans) | in Puffer pH 3,5 |
| aus Aspergillus niger | in Puffer pH 4,0 |
| aus Escherichia coli | in Puffer pH 6,9 |
| aus Rinderleber | in Puffer pH 7,3 |

Die Aktivität dieser Lösungen beträgt ca. 0,08 U/ml (bezogen auf die Hersteller-Angaben).

b) Durchführung der Messungen:

Die Enzymreaktion erfolgt während einer definierten Reaktionszeit bei dem für das jeweilige Enzym optimalen pH-Wert. Es werden 1000 µl Reagens in eine 1-cm-Küvette bei 37 °C mit 30 µl einer Enzymlösung versetzt. Nach 15 Minuten Reaktionszeit wird mit Natronlauge auf pH 8,5 eingestellt und die Extinktion bei 578 nm ermittelt. Die Temperatur wird während der Reaktion und der Messung bei 37 °C konstant gehalten. In gleicher Weise wird zu jeder Messung eine Blindprobe durchgeführt. Hierzu wird statt der Enzymlösung 30 µl Pufferlösung eingesetzt.

c) Auswertung:

Zunächst wird die Differenz zwischen Meßwert mit Enzym und Meßwert der Blindprobe gebildet. Durch Division dieser Differenz durch die Reaktionszeit wird als Maß für die Reaktion der Extinktionsanstieg pro Zeiteinheit in [mExt/min] ermittelt.
Meßwert (mit Enzym) - Meßwert (Blindprobe) = Δ-Meßwert

$$\frac{\Delta\text{-Meßwert}}{\text{Reaktionszeit}} = \text{Reaktion [mExt/min]}$$

Die gefundenen Meßwerte für die Reaktion der einzelnen Enzyme sind der folgenden Zusammenstellung zu entnenmen:

| Enzym aus | pH-Wert | Reaktionsgeschwindigkeit [mExt/min] |
|---|---|---|
| Bohnen (Jack Beans) | 3,5 | 71 |
| Aspergillus Niger | 4,0 | 112 |
| Escherichia coli | 6,9 | 74 |
| Rinderleber | 7,3 | 32 |

Vorstehend beschriebene Versuchsergebnisse zeigen, daß die Sulfonphthaleinyl-β-D-galactoside als Substrate für β-D-Galactosidasen jeder Herkunft geeignet sind.

Beispiel 15

Bestimmung der Aktivität freier und konjugierter β-D-Galactosidase

a) Herstellung der verwendeten Lösungen

| | | |
|---|---|---|
| Pufferlösung: | HEPES | 100 mmol/l |
| | Natriumchlorid | 154 mmol/l |
| | Magnesium-L-Aspartat | 2 mmol/l |
| | BSA | 10 g/l |
| | Tween-20 | 0,5 g/l |
| | pH-Wert (mit Natronlauge eingestellt) | 7,3 (37 °C) |

Reagenslösung:

In vorstehend beschriebener Pufferlösung werden 5 mmol/l 3,3'-Dichlor-phenolsulfonphthaleinyl-β-D-galactosidnatriumsalz gelöst. Der pH-Wert der Pufferlösung mit 7,3 (37 °C) wird beibehalten.

Enzymlösung:

Handelsübliche β-D-Galactosidase aus Escherichia coli wird in Puffer gelöst. Die Aktivität dieser Lösung beträgt ca. 0,08 U/ml (bezogen auf die Hersteller-Angaben).

Enzym-Konjugat-Lösung:

Verwendet wird ein β-D-Galactosidase-Antikörper-Präparat. Die Herstellung solcher Enzym-Antikörper-Konjugate ist bekannt. Sie ist beispielsweise in Biochem. Biophys. Acta 612, 40 - 49 (1980) beschrieben. Das Präparat wird in Puffer so verdünnt, daß sich eine mit der oben beschriebenen Enzymlösung ungefähr vergleichbare Aktivität ergibt.

b) Durchführung der Messung:

Die Messung erfolgt photometrisch bei 578 nm. 950 µl Reagenslösung werden jeweils in einer 1-cm-Küvette bei 37 °C mit 50 µl Enzymlösung bzw. mit 50 µl Enzym-Konjugat-Lösung vermischt. Als Maß für die Reaktion wird der Extinktionsanstieg pro Zeiteinheit in [mExt/min] ermittelt.

Für die Reaktion mit der freien β-D-Galactosidase werden 124 mExt/min; für die Reaktion mit β-D-Galactosidase-Antikörper-Konjugat 120 mExt/min gemessen.

Beide Meßwerte zeigen, daß sowohl mit freier als auch mit konjugierter β-D-Galactosidase ein sehr gut meßbarer Extinktionsunterschied gefunden wird. Daraus ergibt sich, daß die Sulfonphthaleinyl-β-D-galactoside als Substrate sowohl für freie β-D-Galactosidase als auch für β-D-Galactosidase-Konjugate in gleicher Weise geeignet sind. Die neuen Substrate können somit nicht nur als diagnostische Mittel für die Bestimmung freier β-D-Galactosidasen eingesetzt werden. Sie sind in vorteilhafter Weise auch bei Enzymimmunoassays einsetzbar, bei denen β-D-Galactosidase als Indikator-Enzym verwendet wird.

## Ansprüche

**1.** Phenolsulfonphthaleinyl-β-D-galactoside der allgemeinen Formel I

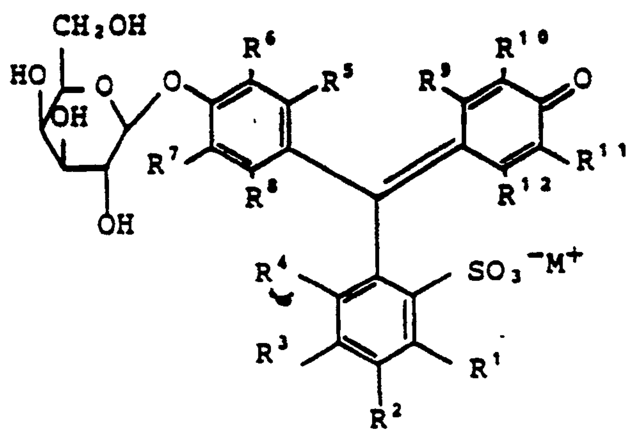

(I)

in der

| | |
|---|---|
| $R^1$ bis $R^4$, | die gleich oder verschieden sein können, jeweils Wasserstoff, Halogen, eine Nitro- oder eine Aminogruppe, |
| $R^5$ bis $R^{12}$, | die gleich oder verschieden sein können, jeweils Wasserstoff, Halogen, eine $(C_1$-$C_5)$-Alkyl-, eine Hydroxy-, eine $(C_1$-$C_5)$-Alkoxy-, eine Carboxyl- oder eine Nitrogruppe, |
| $M^+$ | ein Proton, ein Alkali-, ein Erdalkali- oder ein Ammoniumion |

bedeuten.

2. Phenolsulfonphthaleinyl-β-D-galactosid gemäß Anspruch 1 aus der Gruppe

Phenolsulfonphthaleinyl-β-D-galactosid,
3,3'-Difluor-phenolsulfonphthaleinyl-β-D-galactosid,
3,3'-Dichlor-phenolsulfonphthaleinyl-β-D-galactosid und
3,3',5,5'-Tetrachlor-3",4",5",6"-tetrabrom-phenolsulfonphthaleinyl-β-D-galactosid.

3. Verfahren zur Herstellung der Phenolsulfonphthaleinyl-β-D-galactoside der allgemeinen Formel I

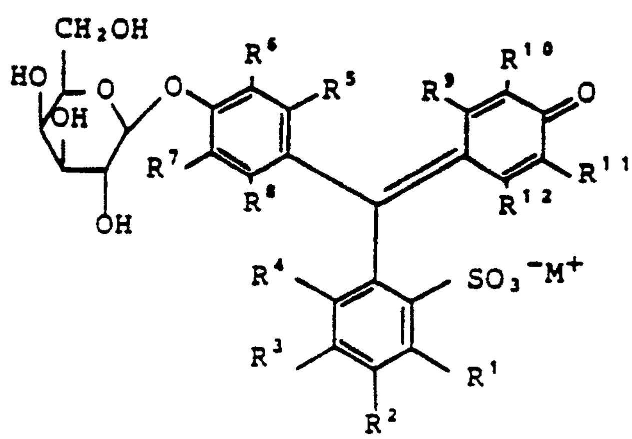

(I)

in der

| | |
|---|---|
| $R^1$ bis $R^4$, | die gleich oder verschieden sein können, jeweils Wasserstoff, Halogen, eine Nitro- oder eine Aminogruppe, |
| $R^5$ bis $R^{12}$, | die gleich oder verschieden sein können, jeweils Wasserstoff, Halogen, eine $(C_1$-$C_5)$-Alkyl-, eine Hydroxy-, eine $(C_1$-$C_5)$-Alkoxy-, eine Carboxyl- oder eine Nitrogruppe, |
| $M^+$ | ein Proton, ein Alkali-, ein Erdalkali- oder ein Ammoniumion |

bedeuten, dadurch gekennzeichnet, daß man in an sich bekannter Weise Verbindungen der allgemeinen Formel II

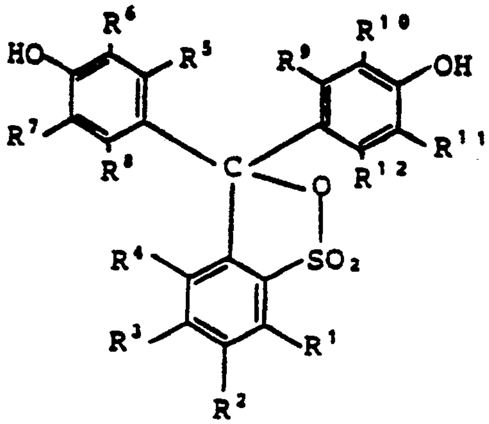

(II)

in der $R^1$ bis $R^{12}$ die oben angegebene Bedeutung haben,

mit per-0-substituierter 1-Halogeno-α-D-galactose der allgemeinen Formel III

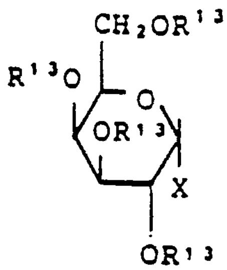

(III)

in der X Halogen und $R^{13}$ eine in der Kohlenhydratchemie gebräuchliche Schutzgruppe

bedeuten,

unter Walden-Umkehr am C-1-Atom des Zuckerrestes zu per-0-substituierten Sulfonphthaleinyl-β-D-galactosiden der allgemeinen Formel IV

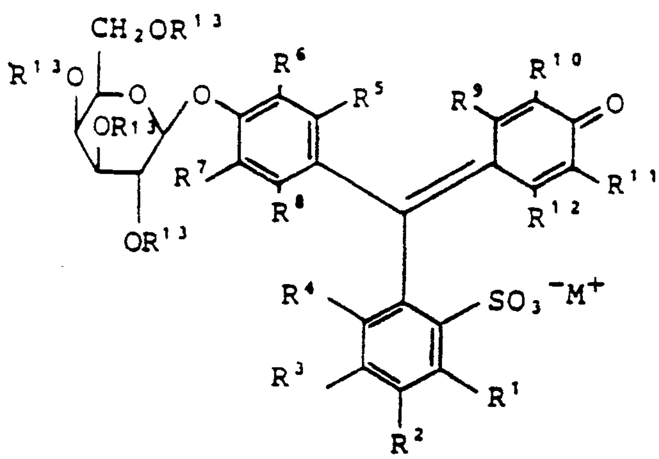

(IV)

umsetzt und von letzteren in an sich bekannter Weise die Schutzgruppen $R^{13}$ abspaltet.

**4.** Verwendung von Phenolsulfonphthaleinyl-β-D-galactosiden gemäß Anspruch 1 oder 2 zur Bestimmung der Aktivität von β-D-Galactosidase.

5. Diagnostisches Mittel zum Nachweis der β-D-Galactosidase, enthaltend ein oder mehrere chromogene Substrate, eine geeignete Puffersubstanz sowie gegebenenfalls weitere üblicherweise verwendete Hilfsstoffe, dadurch gekennzeichnet, daß als chromogene Substrate Phenolsulfonphthaleinyl-β-D-galactoside gemäß Anspruch 1 oder 2 eingesetzt werden.

6. Diagnostisches Mittel gemäß Anspruch 5, dadurch gekennzeichnet, daß als zusätzliche Hilfsmittel Netzmittel, Oxidationsmittel, galenische Zusatzstoffe und/oder Gerüstbildner verwendet werden.

## Claims

1. Phenolsulphonphthaleinyl-β-D-galactosides of the general formula I

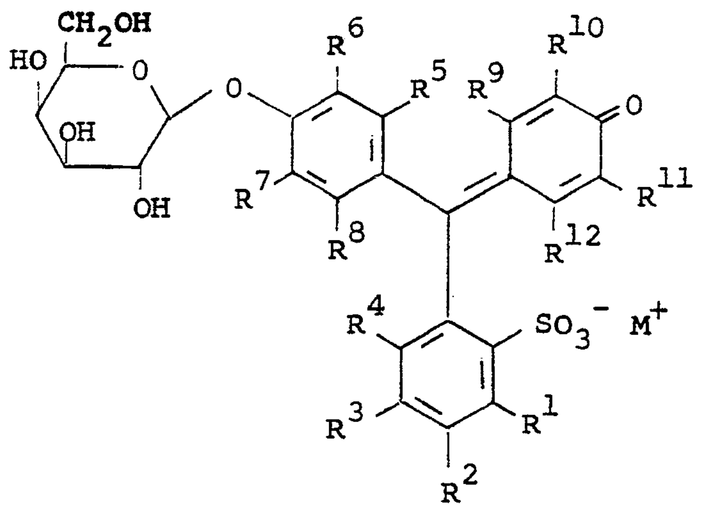

(I)

in which $R^1$ to $R^4$, which can be the same or different, signify, in each case, hydrogen, halogen, a nitro or an amino group, $R^5$ to $R^{12}$, which can be the same or different, signify, in each case, hydrogen, halogen, a $(C_1-C_5)$-alkyl, a hydroxyl, a $(C_1-C_5)$-alkoxy, a carboxyl or a nitro group, $M^+$ signifies a proton, an alkali metal, an alkaline earth metal or an ammonium ion.

2. Phenolsulphonphthaleinyl-β-D-galactoside according to claim 1 from the group phenolsulphonphthaleinyl-β-D-galactoside, 3,3'-difluorophenolsulphonphthaleinyl-β-D-galactoside, 3,3'-dichlorophenolsulphonphthaleinyl-β-D-galactoside and 3,3',5,5'-tetrachloro-3",4",5",6"-tetrabromophenolsulphonphthaleinyl-β-D-galactoside.

3. Process for the preparation of phenolsulphonphthaleinyl-β-D-galactosides of the general formula I

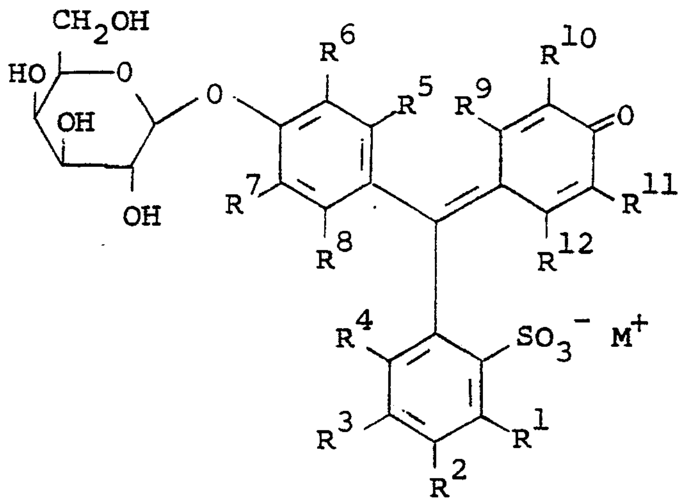

(I)

in which $R^1$ to $R^4$, which can be the same or different, signify, in each case, hydrogen, halogen, a nitro or an amino group, $R^5$ to $R^{12}$, which can be the same or different, signify, in each case, hydrogen, halogen, a $(C_1$-$C_5)$-alkyl, a hydroxyl, a $(C_1$-$C_5)$-alkoxy, a carboxyl or a nitro group, $M^+$ signifies a proton, an alkali metal, an alkaline earth metal or an ammonium ion, characterised in that, in per se known manner, one reacts compounds of the general formula II

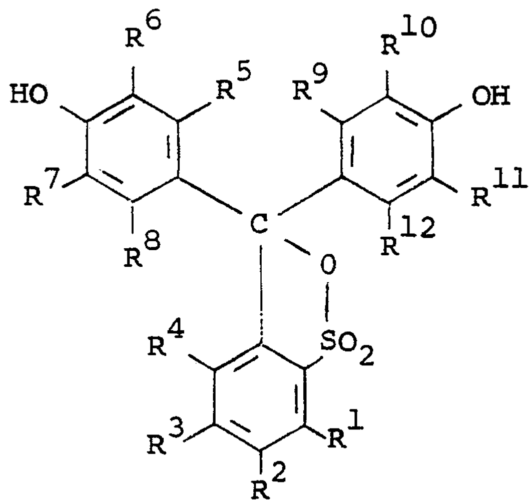

(II)

in which $R^1$ to $R^{12}$ have the above-given meaning, with per-0-substituted 1-halo-α-D-galactoses of the general formula III

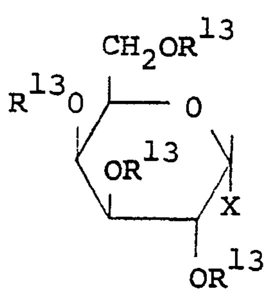

(III)

in which X signifies halogen and $R^{13}$ a protective group conventional in carbohydrate chemistry, with Walden inversion on the C-1 atom of the sugar residue to give per-0-substituted sulphonphthaleinyl-β-D-galactosides of the general formula IV

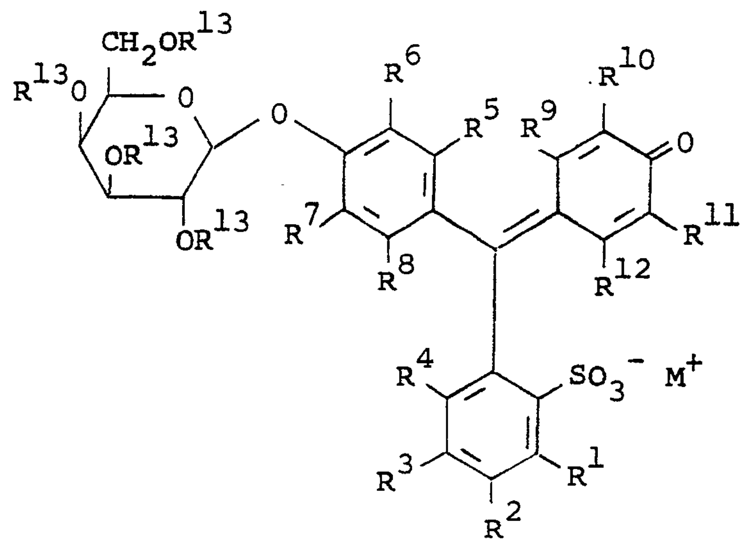

(IV)

and splits off the protective groups $R^{13}$ from the latter in per se known manner.

4. Use of phenolsulphonphthaleinyl-β-D-galactosides according to claim 1 or 2 for the determination of the activity of β-D-galactosidase.

5. Diagnostic agent for the detection of β-D-galactosidase, containing one or more chromogenic substrates, a suitable buffer substance, as well as possibly further conventionally used adjuvants, characterised in that, as chromogenic substrates, there are used phenolsulphonphthaleinyl-β-D-galactosides according to claim 1 or 2.

6. Diagnostic agent according to claim 5, characterised in that, as additional adjuvants, there are used wetting agents, oxidation agents, galenical adjuvants and/or structure formers.

**Revendications**

1. Phénolsulfonephtaléinyl-β-D-galactosides de formule générale I

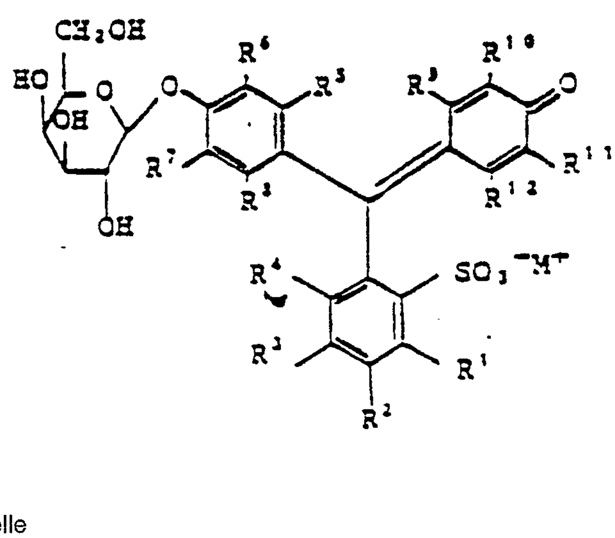

(I)

dans laquelle

$R^1$ à $R^4$, qui peuvent être identiques ou différents, représentent chacun un atome d'hydrogène, un atome d'halogène, un groupe nitro ou un groupe amino,

$R^5$ à $R^{12}$, qui peuvent être identiques ou différents, représentent chacun un atome d'hydrogène, un atome d'halogène, un groupe alkyle en $C_1$-$C_5$, un groupe hydroxy, un groupe alcoxy en $C_1$ -$C_5$, un groupe carboxyle ou un groupe nitro,

$M^+$ représente un proton, un ion alcalin, alcalinoterreux ou ammonium.

2. Phénolsulfonephtaléinyl-$\beta$-D-galactosides selon la revendication 1, du groupe se composant des

Phénolsulfonephtaléinyl-$\beta$-D-galactoside,
3,3'-Difluorophénolsulfonephtaléinyl-$\beta$-D-galactoside,
3,3'-Dichlorophénolsulfonephtaléinyl-$\beta$-D-galactoside, et
3,3',5,5'-Tétrachloro-3",4",5",6"-tétrabromophénolsulfonephtaléinyl-$\beta$-D-galactoside.

3. Procédé pour la préparation des phénolsulfonephtaléinyl-$\beta$-D-galactosides de formule générale I

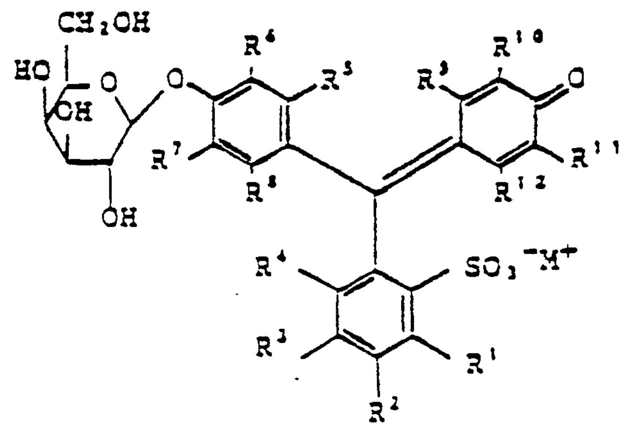

(I)

dans laquelle

$R^1$ à $R^4$, qui peuvent être identiques ou différents, représentent chacun un atome d'hydrogène, un atome d'halogène, un groupe nitro ou un groupe amino,

$R^5$ à $R^{12}$, qui peuvent être identiques ou différents, représentent chacun un atome d'hydrogène, un atome d'halogène, un groupe alkyle en $C_1$-$C_5$, un groupe hydroxy, un groupe alcoxy en $C_1$ -$C_5$, un groupe carboxyle ou un groupe nitro,

$M^+$ représente un proton, un ion alcalin, alcalinoterreux ou ammonium,

caractérisé en ce que de manière connue en soi, on fait réagir des composés de formule générale II

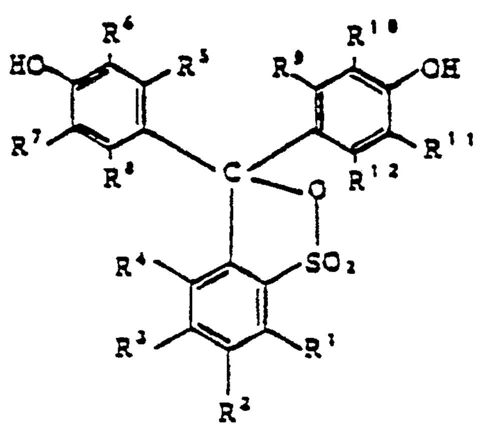

(II)

dans laquelle $R^1$ à $R^{12}$ ont les significations mentionnées ci-dessus, avec des 1-halogéno-$\alpha$-D-galactoses per-O-substitués, de formule générale III

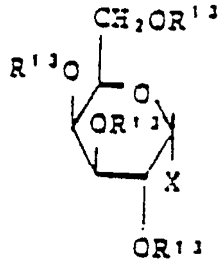

(III)

dans laquelle X désigne un atome d'halogène et $R^{13}$ un groupe protecteur usuel dans la chimie des hydrates de carbone, avec une inversion de Walden sur l'atome C-1 du reste sucre, en sulfonephtaléinyl-β-D-galactosides per-O-substituées de formule générale IV

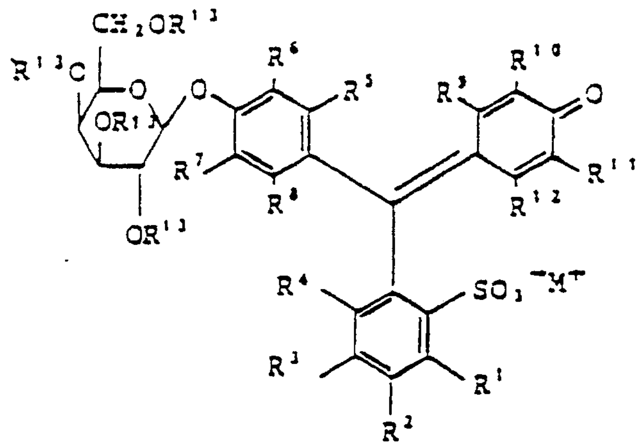

(IV)

et que l'on sépare le groupe protecteur $R^{13}$ de ces derniers composés, de manière connue en soi.

4. Utilisation des phénolsulfonephtaléinyl-β-D-galactosides selon la revendication 1 ou 2, pour la détermination de l'activité de β-D-galactosidases.

5. Agent de diagnostic pour la détection de β-D-galactosidases, contenant un ou plusieurs substrats chromogènes, une substance tampon appropriée ainsi qu'éventuellement d'autres adjuvants usuels, caractérisé en ce que comme substrat chromogène, on utilise des phénolsulfonephtaléinyl-β-D-galactosides selon la revendication 1 ou 2.

6. Agent de diagnostic selon la revendication 5, caractérisé en ce que comme adjuvant supplémentaire, on utilise des agents mouillants, des agents oxydants, des additifs galéniques et/ou des formateurs de squelette.